# EUROPEAN PATENT SPECIFICATION

(11) **EP 4 085 954 B1**
(45) Date of publication and mention of the grant of the patent: **19.03.2025**
(21) Application number: 19958324.6
(22) Date of filing: 30.12.2019
(51) Int. Cl.: A61M 16/01, A61G 12/00

(54) **ANESTHESIA MACHINE**
ANÄSTHESIEGERÄT
MACHINE D'ANESTHÉSIE

(43) Date of publication of application: 09.11.2022
(73) Proprietor: Shenzhen Mindray Bio-Medical Electronics Co., Ltd., Shenzhen, Guangdong 518057 (CN)
(72) Inventor: YANG, Zhanxiao, Shenzhen, Guangdong 518057 (CN); LUO, Jiancheng, Shenzhen, Guangdong 518057 (CN); CHEN, Peitao, Shenzhen, Guangdong 518057 (CN)
(74) Representative: KIPA AB
(86) International application number: PCT/CN2019/130213
(87) International publication number: WO 2021/134372

(56) References cited:
- WO-A2-2014/178730
- CN-A- 104 815 375
- CN-A- 106 178 211
- CN-A- 106 693 135
- CN-A- 109 069 782
- CN-A- 109 069 782
- CN-A- 109 731 200
- CN-U- 205 434 615
- CN-U- 206 424 397
- GB-A- 255 303
- US-A1- 2005 005 932
- US-A1- 2012 180 789
- US-A1- 2017 209 658

## Description

### TECHNICAL FIELD

The disclosure relates to the technical field of medical devices, and in particular to an anesthesia machine.

### BACKGROUND

An anesthesia machine has the functions of inhalation anesthesia and mechanical ventilation for a patient during a surgery. The patient is assisted by an anesthesia ventilator to maintain airway patency, improve ventilation and oxygenation, and prevent body hypoxia and the accumulation of carbon dioxide.

However, in order to ensure the normal operation of the components inside the anesthesia machine, a fan is usually used for forced heat dissipation, which generates noise during the operation of the fan, and causes an adverse effect on an operating room environment. Patent applications CN109069782 and US2017209658 provide teachings related to the technical field of the application.

### SUMMARY

The disclosure provides an anesthesia machine, which is capable of preventing heat generated by a main control board from affecting other components inside a host case, and also ensures that the main control board has a sufficient heat dissipation space, so as to prolong the service life of the main control board and other components inside the anesthesia machine.

The disclosure provides an anesthesia machine, including a gas source interface, an anesthesia ventilation circuit, an anesthetic evaporator, a host case, a main control board, a display apparatus and a power supply interface;
the host case comprises a main rack case and a main control case arranged above the main rack case;
the gas source interface and the power supply interface are arranged on the host case, and the anesthetic evaporator is detachably connected to the host case;
the anesthesia ventilation circuit is connected to the gas source interface, and at least part of the anesthesia ventilation circuit is arranged in the host case;
the display apparatus includes a display device and a display shell for fixing the display device;
the display apparatus is installed at an upper end of the main control case by means of a display supporting frame and is arranged separately from the main control case; and
the main control board is arranged on a side of the display shell opposite to the display device, and the power supply interface is electrically connected to the main control board and the display device.

**In** the anesthesia machine of the disclosure, the gas source interface and the power supply interface are separately arranged.

In the anesthesia machine of the disclosure, a control panel is arranged on a front side of the host case for a user to adjust parameter information of the anesthesia ventilation circuit and/or display control parameters of the anesthesia ventilation circuit.

In the anesthesia machine of the disclosure, the anesthetic evaporator is arranged on a left side or a right side of the control panel, and the gas source interface and the power supply interface are arranged on a back side of the host case.

The anesthesia machine of the disclosure further includes:
a control board configured to control power supply among various components on the anesthesia machine, where the control board is arranged inside the host case.

In the anesthesia machine of the disclosure, the control board is arranged on a back side of the host case, and the control board is arranged below the power supply interface.

The anesthesia machine of the disclosure further includes:
a power supply apparatus arranged on a bottom of an inner side of the host case or on a base below the host case, the power supply apparatus being arranged separately from the control board and the control panel.

In the anesthesia machine of the disclosure, the control panel is arranged on the main control case.

In the anesthesia machine of the disclosure, the gas source interface is arranged on the main rack case, and/or the power supply interface is arranged on the main control case.

In the anesthesia machine of the disclosure, an upper surface of the main rack case forms a worktable top, and the anesthetic evaporator is detachably placed on the worktable top.

In the anesthesia machine of the disclosure, the power supply interface includes:
an auxiliary output interface; and
a power input interface arranged on one side of the auxiliary output interface.

In the anesthesia machine of the disclosure, the control board is arranged on a back side of the main control case, and the auxiliary output interface and the power input interface are both arranged above the control board.

In the anesthesia machine of the disclosure, the control board is arranged in the main rack case; or the control board is arranged in the main control case.

The anesthesia machine of the disclosure further includes:
an AGSS module arranged on a side of the host case, the AGSS module being in communication with the anesthesia ventilation circuit.

The anesthesia machine of the disclosure further includes:
a plug-in module interface arranged on the host case.

In the anesthesia machine of the disclosure, the plug-in module interface and the AGSS module are located on the same side.

In the anesthesia machine of the disclosure, the anesthesia ventilation circuit includes a breathing circuit, a driving gas branch and a fresh gas branch, where the fresh gas branch is arranged on the inner side of the host case, and the driving gas branch and the breathing circuit are arranged on an outer side of the host case.

In the anesthesia machine of the disclosure, the anesthesia ventilation circuit further includes:
a pressure detection assembly configured to detect a gas pressure of the anesthesia ventilation circuit, where the pressure detection assembly, the control panel and the control board are separately arranged on the host case.

In the anesthesia machine of the disclosure, the pressure detection assembly is arranged in the main control case, or the pressure detection assembly is arranged at a position of the main rack case on the worktable top.

In the anesthesia machine of the disclosure, a heat dissipation structure is arranged on the display shell, and the heat dissipation structure corresponds to the main control board in position.

In the anesthesia machine of the disclosure, the heat dissipation structure includes a heat conducting pad, one end of the heat conducting pad abutting against the main control board, and the other end of the heat conducting pad abutting against the display shell.

The technical solutions provided in the embodiments of the disclosure may have the following beneficial effects: the disclosure designs an anesthesia machine, since the main control board is arranged on the display shell, the heat generated by the main control board is prevented from affecting other components inside the host case, especially some components which operate only at a lower temperature, thereby providing a safe operating temperature for each component, ensuring that each component in the anesthesia machine is capable of operating normally within a specified temperature range thereof, and then ensuring the use safety and service life of the anesthesia machine.

It should be understood that the above general description and the following detailed description are only exemplary and explanatory, and cannot limit the disclosure.

### BRIEF DESCRIPTION OF THE DRAWINGS

In order to more clearly describe the technical solutions in the embodiments of the disclosure, the drawings required for describing the embodiments will be briefly described below. Apparently, the drawings in the following description show some of the embodiments of the disclosure, and persons of ordinary skill in the art may still derive other drawings from these drawings without creative efforts.
FIG. 1 is a schematic structural diagram of an anesthesia machine provided in an embodiment of the disclosure;
FIG. 2 is a schematic diagram of the anesthesia machine of FIG. 1 at another angle;
FIG. 3 is a schematic diagram of the anesthesia machine of FIG. 1 at another angle;
FIG. 4 is a schematic diagram of the anesthesia machine of FIG. 1 at another angle; and
FIG. 5 is a schematic diagram of the anesthesia machine of FIG. 1 at another angle.

### List of reference numerals:

10. Host case; 11. Main rack case; 111. Worktable top; 112. Base; 12. Main control case; 20. Display apparatus; 21. Display device; 22. Display shell; 30. Anesthetic evaporator; 40. Control panel; 50. Breathing circuit; 51. Carbon dioxide absorption tank; 60. Main control board; 70. Control board; 80. Power supply interface; 81. Auxiliary output interface; 82. Power input interface; 90. Gas source interface; 100. Pressure detection assembly; 110. AGSS module; 120. Plug-in module interface; 130. Power supply apparatus.

### DESCRIPTION OF THE PREFERRED EMBODIMENTS

The technical solutions of the embodiments of the disclosure will be described below clearly and comprehensively in conjunction with accompanying drawings of the embodiments of the disclosure. Apparently, the embodiments described are some of, rather than all of, the embodiments of the disclosure. All other embodiments obtained by persons of ordinary skill in the art based on the embodiments of the disclosure without creative efforts shall fall within the scope of protection of the disclosure.

In the description of the disclosure, it should be understood that the orientation or position relationship indicated by the terms "centre", "longitudinal", "transverse", "length", "width", "thickness", "upper", "lower", "front", "rear", "left", "right", "vertical", "horizontal", "top", "bottom", "inner", "outer", "clockwise", "counterclockwise", etc. are based on the orientation or position relationship shown in the accompanying drawings and are intended to facilitate the description of the disclosure and simplify the description only, rather than indicating or implying that the apparatus or element referred to must have a particular orientation or be constructed and operated in a particular orientation, and therefore cannot be understood as limiting the disclosure. In addition, the terms "first" and "second" are used for descriptive purposes only, and cannot be construed as indicating or implying relative importance or implicitly indicating the number of technical features indicated. Thus, the features defined with "first" and "second" may explicitly or implicitly include one or more of the features. In the description of the disclosure, the meaning of "a plurality of" is two or more, unless specifically and specifically limited otherwise.

Some embodiments of the disclosure will be described in detail below with reference to the drawings. In the case of no conflict, the embodiments and the features thereof described below may be combined with each other.

The anesthesia machine of the disclosure belongs to the technical field of medical devices. An anesthesia machine is one of the most commonly used devices in an operating room and is a medical device mainly for adjusting a consciousness level and a pain level of a patient during a surgery.

In general, the anesthesia machine includes a gas supply part, a control part, an anesthesia ventilation circuit part, a waste gas discharging part, an anesthetic evaporator, and so on, where the control part includes a control panel, a power input module, an auxiliary output module, a gas pressure detection module, an anesthetic evaporator control module, a plug-in module, an AGSS module and a display module, and so on; and these electronic modules may generate heat during the operation of the anesthesia machine, and when the heat is accumulated too much, it is likely to cause failures of electronic or gas circuit components, and even cause risks of fire, etc.

At present, the anesthesia machine is generally provided with a heat dissipation hole and a cooling fan at a position where heat accumulates, so as to forcibly blow out the heat inside the anesthesia machine and introduce cold air, enabling each component in the anesthesia machine to work normally within a specified temperature range thereof. However, the cooling fan not only has an ineffective gap, but also easily generates noise during operation, which causes an adverse effect on an operating room environment.

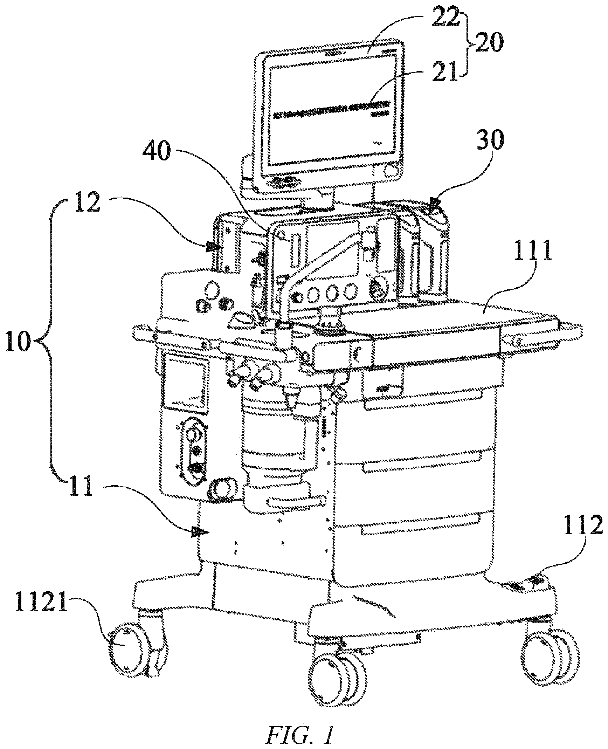

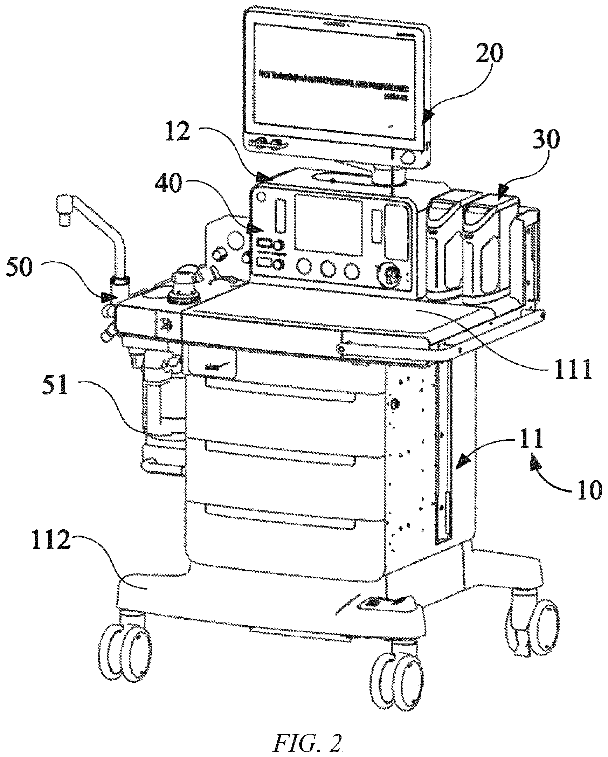

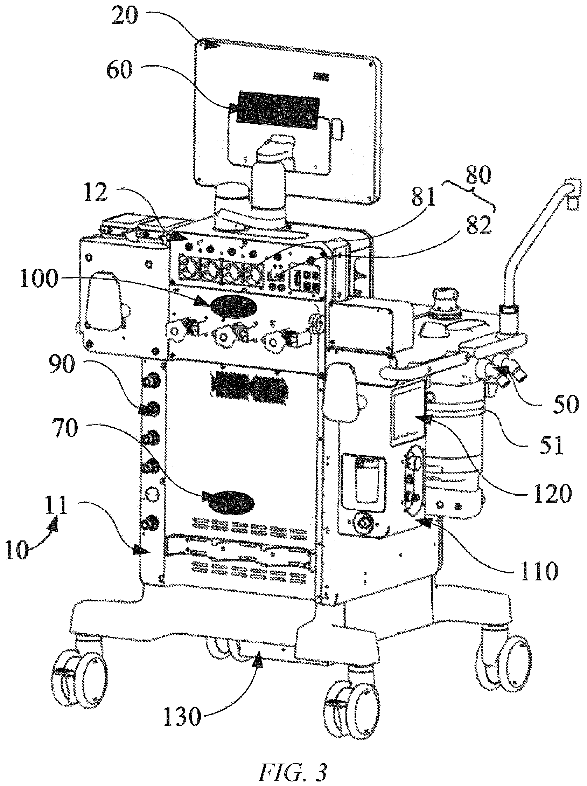

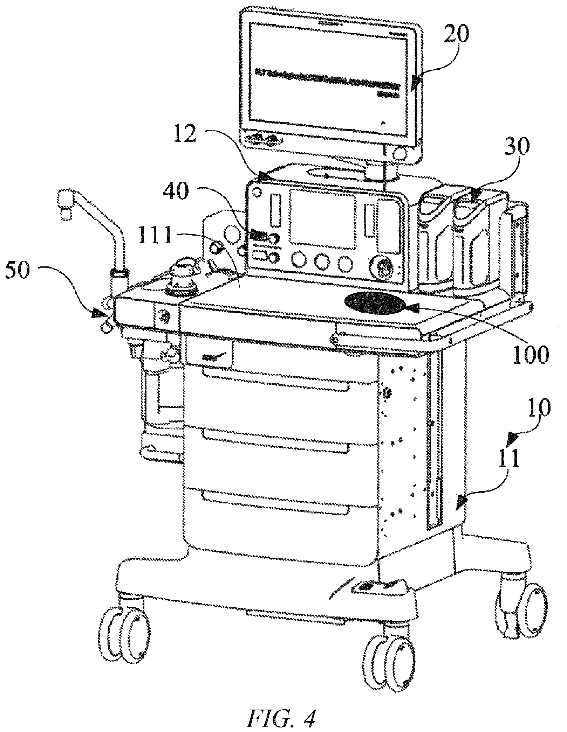

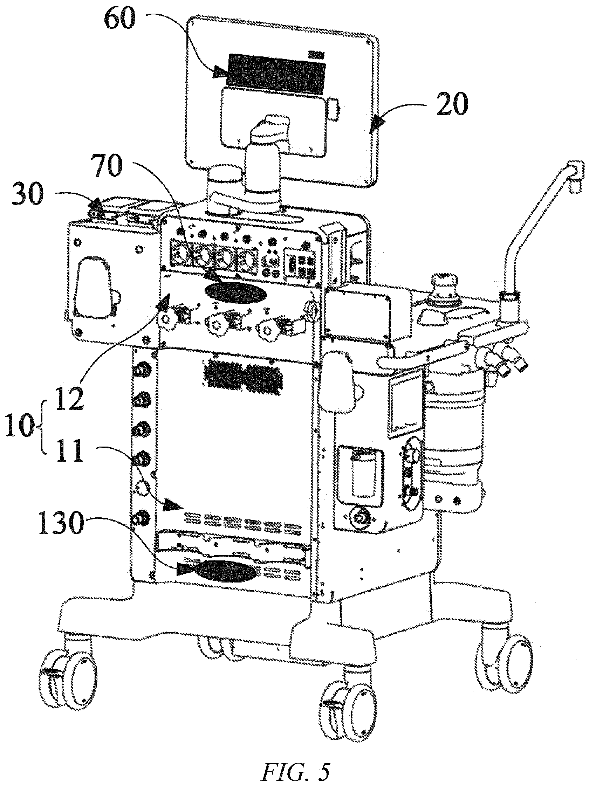

As shown in FIGS. 1-5, the disclosure provides an anesthesia machine, including a host case 10, a display apparatus 20, an anesthetic evaporator 30, an anesthesia ventilation circuit, a main control board 60, a power supply interface 80 and a gas source interface 90, where the gas source interface 90 and the power supply interface 80 are both arranged on the host case 10, the anesthetic evaporator 30 is detachably connected to the host case 10, the gas source interface 90 is connected to the anesthesia ventilation circuit, and at least part of the anesthesia ventilation circuit is arranged in the host case 10. In this embodiment, the display apparatus 20 includes a display device 21 and a display shell 22 for fixing the display device 21, the main control board 60 is arranged on the display shell 22, and the main control board 60 is electrically connected to the power supply interface 80 and the display device 21.

After the above technical solution is used, during the operation of the anesthesia machine, the amounts of heat generated by components inside the anesthesia machine are different. For example, the main control board 60 is a component with a large amount of heat itself, while a control valve on the anesthesia ventilation circuit is a component with a small amount of heat or no heat; when the components such as the main control board 60 and the anesthesia ventilation circuit are integrated in the host case 10, interference will occur between the components such as the main control board 60 and the anesthesia ventilation circuit, and the component releasing a large amount of heat will affect the component with a small amount of heat or no heat, thereby shortening the service life of the component with a small amount of heat or no heat. Also, arranging the main control board 60 with a large amount of heat on the display shell 22 in the disclosure may not only prevent the heat generated by the main control board 60 during operation from affecting the operation of each component in the host case 10, but also ensure that each component in the host case 10 is capable of operating at a safe operating temperature; furthermore, heat dissipation of the main control board 60 is facilitated, a sufficient heat dissipation space is provided for the main control board 60 to ensure that the main control board 60 is capable of dissipating heat to the surroundings by means of the display shell 22.

In an optional embodiment, the main control board 60 may be arranged on an inner side of the display shell 22, and the main control board 60 may also be arranged on an outer side of the display shell 22. When the main control board 60 is arranged on the outer side of the display shell 22, the main control board 60 may be installed in a box and then the box is fixed to the outer side of the display shell 22, and the shape and size of the box may match the shape and size of the display shell 22 to ensure a structural shape of the exterior of the anesthesia machine. Meanwhile, a groove for placing the main control board 60 may also be provided on the outer side of the display shell 22, and is then covered with a corresponding cover to protect the main control board 60, etc. This arrangement is mainly intended to arrange the main control board 60 on the display shell 22, but a fixation method therefor is not limited in the disclosure.

In an optional embodiment, a heat dissipation structure is arranged on the anesthesia machine, the heat dissipation structure is installed on the display shell 22, and the heat dissipation structure corresponds to the main control board 60 in position, such that the heat generated by the main control board 60 during operation can be rapidly diffused into the air by means of the heat dissipation structure, achieving a cooling effect.

Specifically, the heat dissipation structure may include an air intake hole and an air outlet hole formed in the display shell 22, where the air intake hole and the air outlet hole are in communication to form a cooling air channel, and the cooling air channel runs through an outer surface of the main control board 60; when the anesthesia machine operates, the heat generated by the main control board 60 due to the operation can be released into the air by means of an air flow in the cooling air channel, ensuring that the main control board 60 can operate at a specified temperature, and prolonging the service life of the main control board 60.

In order to improve the temperature reduction and cooling efficiency of the display shell 22, the display shell 22 may also be provided with a miniature cooling fan, and the cooling fan can accelerate the discharge of a hot air in the display shell 22 without increasing the noise of the anesthesia machine, nor causing an adverse effect on an operating room environment.

In addition, the heat dissipation structure may further include a heat sink, where the heat sink is closely adhered to the main control board 60, maximally increasing the heat dissipation area of the main control board 60, so that electronic elements on the main control board 60 sufficiently dissipate heat. The heat sink may be a heat dissipation fin made of an aluminum alloy material, and the size and shape thereof may be the size and shape of the main control board 60, such that the heat dissipation effect of the heat dissipation structure can be effectively increased.

In an optional embodiment, the heat dissipation structure includes a heat conducting pad, where one end of the heat conducting pad abuts against the main control board 60, and the other end of the heat conducting pad abuts against the display shell 22, such that the heat generated during the operation of the main control board 60 can be transferred to the display shell 22 by means of the heat conducting pad, and then diffused into the air by means of the display shell 22; alternatively, the inner side of the display shell 22 is provided with a metal frame, and the heat conducting pad is connected to the metal frame and diffuses the heat generated during the operation of the main control board 60 into the air by means of the metal frame, etc.

After the above technical solution is used, since the heat dissipation structure is arranged on the display shell 22, the heat dissipation structure can diffuse the heat generated during the operation of the main control board 60 into the air, and can discharge the heat inside the display shell 22 to the outside air, such that the heat dissipation effect of the main control board 60 is more obvious, ensuring that the main control board 60 can operate at the specified temperature thereof, and prolonging the service life of the main control board 60.

In an optional embodiment, the gas source interface 90 and the power supply interface 80 are arranged separately to achieve gas-electricity separation, which can effectively and completely eradicate the contact between the anesthesia ventilation circuit and various components and/or cables, and avoid the occurrence of relevant safety accidents, so that the safety factor of the host case 10 is higher, and the safety in use is ensured. Meanwhile, it also facilitates the arrangement of pipelines on the anesthesia ventilation circuit and the cables connected among the various components in the host case 10. For example, since the gas source interface 90 and the power supply interface 80 are respectively arranged on two sides of the host case 10, the pipelines on the anesthesia ventilation circuit may be arranged along one side of the host case 10, and the cables connected among the various components in the host case 10 may be arranged along the other side of the host case 10, avoiding the problem of entanglement of the cables and the pipelines crossing each other, which not only facilitates the orderly arrangement of the pipelines and the cables inside the host case 10, but also avoids the influence of the heat generated by the cables during power supply on the pipelines, preventing the service life of the pipelines from being shortened. The embodiment has the characteristics of reasonable layout, convenient installation, quick maintenance, cost saving, etc.

In an embodiment not being part of the invention, the display apparatus 20 may be directly arranged on a front side of the host case 10. In an embodiment, the display apparatus 20 is installed at an upper end of the host case 10 by means of a display supporting frame. In this embodiment, the display apparatus 20 is installed at the upper end of the host case 10 by means of the display supporting frame, and the main control board 60 is arranged on the side of the display shell 22 opposite to the display device 21, such that heat generated during the operation of the main control board 60 can be rapidly diffused into the air by means of the display shell 22 to play a cooling effect.

In an optional embodiment, the front side of the host case 10 is provided with a control panel 40, and the control panel 40 is used for a user to adjust parameter information of the anesthesia ventilation circuit and/or to display control parameters of the anesthesia ventilation circuit.

Specifically, the control panel 40 includes one or more of a flow adjustment knob, a concentration adjustment knob and a touch screen, where a gas flow parameter in the anesthesia ventilation circuit may be adjusted by means of the flow adjustment knob, a gas concentration parameter and/or an anesthetic concentration parameter in the anesthesia ventilation circuit may be adjusted by means of the concentration adjustment knob, and the gas flow parameter, the gas concentration parameter and the anesthetic concentration parameter adjusted by means of the flow adjustment knob and/or the concentration adjustment knob may be displayed by the touch screen. Furthermore, the touch screen may also adjust one or more of ventilation and anesthesia control parameters, such as gas flow, gas concentration, and anesthetic concentration, and device control parameters; and the control panel 40 may also include a switching apparatus of the device for turning on or off the device.

In an optional embodiment, the anesthetic evaporator 30 is arranged on a left side or a right side of the control panel 40, and the gas source interface 90 and the power supply interface 80 are arranged on a back side of the host case 10, thereby avoiding mutual interference among the anesthetic evaporator 30, the control panel 40, the gas source interface 90 and the power supply interface 80, and effectively controlling the anesthetic evaporator 30, the control panel 40 and the power supply interface 80 to have a sufficient heat dissipation space, so that the space on the anesthesia machine can be effectively used, and thus the anesthetic evaporator 30, the control panel 40 and the power supply interface 80 can be distributed to various regions of the anesthesia machine for heat dissipation.

Specifically, the right side of the control panel 40 is provided with an open installation cavity, the open installation cavity is provided with a locking seat sliding rail and a gas circuit interface connected to the anesthesia ventilation circuit, and after the anesthetic evaporator 30 is installed in the open installation cavity by means of the locking seat sliding rail, the anesthetic evaporator 30 is in communication with the anesthesia ventilation circuit by means of the gas circuit interface, so that when a patient breathes by means of a ventilator connected in the anesthesia ventilation circuit, the anesthetic evaporator 30 generates anesthetic vapor to anesthetize the patient.

In an optional embodiment, the anesthesia machine further includes a control board 70, the control board 70 being configured to control power supply between components on the anesthesia machine, where the control board 70 is arranged inside the host case 10.

Specifically, the control board 70 is electrically connected to the power supply interface 80 to supply power for controlling the various components in the anesthesia machine; the control board 70 may be arranged on a left side of an interior of the host case 10, or the control board 70 may also be arranged on a right side of the interior of the host case 10, or the control board 70 may also be arranged on a rear side of the interior of the host case 10, or even the control board 70 may also be arranged on a front side of the interior of the host case 10, and may be fixed to any one side of the host case 10 in a fixed manner, or fixed in the middle of the host case 10 by means of a supporting frame, which is not limited in the disclosure.

In an optional embodiment, the control board 70 is arranged on the back side of the host case 10, and the control board 70 is arranged below the power supply interface 80, thereby facilitating the arrangement of the cables between the power supply interface 80 and the control board 70, also preventing the control board 70, the control panel 40 and the power supply interface 80 from interfering with one another due to different amounts of heat during operation, ensuring the stability of the operation of the control board 70, the control panel 40 and the power supply interface 80, and prolonging the service life of the control board 70, the control panel 40 and the power supply interface 80.

In an optional embodiment, the anesthesia machine further includes a power supply apparatus 130, the power supply apparatus 130 being arranged on a bottom of an inner side of the host case 10 or on a base 112 below the host case 10, where the power supply apparatus 130 is arranged separately from the control board 70 and the control panel 40 to reduce interference with the control board 70 and the control panel 40 by the power supply apparatus 130, thereby reducing the influence on the service life of the control board 70 and the control panel 40.

In this embodiment, the power supply apparatus 130 is a disposable or rechargeable power storage apparatus. In case of a sudden power failure during the use of an anesthetic, the power supply apparatus 130 may supply power to the components inside the anesthesia machine by means of the control board 70, preventing the accidental power failure from causing harm to the patient. The power supply apparatus 130 is electrically connected to the power supply interface 80 by means of the control board 70, and when the power supply apparatus 130 has insufficient power, the power supply interface 80 can automatically charge the power supply apparatus 130 to ensure that the power supply apparatus 130 is always maintained in a fully charged state; when there is no current input on the power supply interface 80, the control board 70 controls the power supply apparatus 130 to supply power to the components inside the anesthesia machine, thereby preventing the accidental power failure from causing harm to the patient.

In an embodiment, the host case 10 includes a main rack case 11 and a main control case 12, where a lower end of the main rack case 11 is provided with a base 112, and universal wheels are installed on the base 112 to facilitate the movement of the host case 10, making the anesthesia machine more convenient to use. In this embodiment, the main control case 12 is arranged at an upper end of the main rack case 11, the control panel 40 is arranged on the main control case 12, the display apparatus 20 is arranged at an upper end of the main control case 12, and the power supply apparatus 130 is arranged at a bottom end of the main rack case 11 or below the base 112 so as to achieve the distribution of heat generating components in the anesthesia machine and also ensure the heat dissipation effect of the heat generating components. The heat generating components inside the anesthesia machine can not only be distributed scientifically and reasonably in the regions of the anesthesia machine according to heat generating conditions of the heat generating components inside the anesthesia machine, but also facilitate good heat dissipation of the heat generating components inside the anesthesia machine. For example, the power supply apparatus 130 is arranged below the base 112, the main control board 60 is arranged on the display shell 22 at the upper end of the main control case 12, etc.

In an optional embodiment, the gas source interface 90 is arranged on the main rack case 11, and/or the power supply interface 80 is arranged on the main control case 12 to achieve gas-electricity separation and avoid safety accidents caused by gas-electricity cross contact, thus reducing hidden safety hazards. In this embodiment, the gas source interface 90 is arranged on the main rack case 11, the control board 70 and the power supply interface 80 are both arranged on the main control case 12, and the control board 70 is located below the power supply interface 80, so that the main rack case 11 and the main control case 12 can form a gas cavity and a cable cavity that are independent of each other, which not only facilitates the arrangement of cables and pipelines inside the anesthesia machine, but also facilitates the installation and maintenance of the anesthesia machine, saving time and manpower.

In an optional embodiment, an upper surface of the main rack case 11 connected to the main control case 12 forms a worktable top 111, where the anesthetic evaporator 30 is detachably placed on the worktable top 111, and the worktable top is configured to place medical instruments commonly used during a surgery and notes to be recorded by medical personnel in work. In this embodiment, the anesthetic evaporator 30 is detachably placed in the open installation cavity on the right side of the control panel 40, a layered storage cabinet is arranged on the main rack case 11, and the layered storage cabinet is arranged at an upper end of the base 112 to facilitate the storage of surgical supplies and provide assistance for surgery; and the structural design is relatively reasonable, not only facilitating the taking and placement of the anesthetic evaporator 30, but also improving the working efficiency of medical care personnel.

In an optional embodiment, the power supply interface 80 includes an auxiliary output interface 81 and a power input interface 82, the power input interface 82 being arranged on one side of the auxiliary output interface 81, where the auxiliary output interface 81 is configured to establish an electrical connection or a communication connection to an auxiliary device, the auxiliary device including an ultrasonic instrument, etc.; and the power input interface 82 is configured to convert an alternating voltage into an operating voltage of the anesthesia machine.

Specifically, an AC-DC power module and an auxiliary output module are arranged on the anesthesia machine, and the auxiliary output interface 81 is electrically connected to the auxiliary output module, such that the auxiliary device can be electrically connected to the auxiliary output module; and the power input interface 82 is electrically connected to the AC-DC power module, and the AC-DC power module is configured to convert a 220V alternating voltage into a 12v direct voltage specially used for the anesthesia machine. In this embodiment, the auxiliary output interface 81 is arranged on a left side or a rear side of the power input interface 82, which is mainly aimed to facilitate the arrangement of the cables connected to the auxiliary output interface 81 and the power input interface 82, to avoid mutual crossing between the cables and to effectively reduce the loss of use of the cables, and to facilitate maintenance.

In an optional embodiment, the control board 70 is arranged on a back side of the main control case 12, and the auxiliary output interface 81 and the power input interface 82 are both arranged above the control board 70. Specifically, the auxiliary output interface 81 is installed on the auxiliary output interface 81, the power input interface 82 is installed on the AC-DC power module, namely, the auxiliary output interface 81 is installed on the back side of the main control case 12 by means of the auxiliary output module, the power input interface 82 is installed on the back side of the main control case 12 by means of the AC-DC power module, and the control board 70 is arranged below the auxiliary output module and the AC-DC power module, which not only ensures the heat dissipation space of the control board 70, the auxiliary output module and the AC-DC power module, but also facilitates the arrangement of the cables among the control board 70, the auxiliary output module and the AC-DC power module, thus avoiding the crossing problem of the cables and solving the problem of mutual interference of the cables.

In an optional embodiment, the control board 70 is arranged in the main rack case 11; or, the control board 70 is arranged in the main control case 12, so that the control board 70 having a sufficient heat dissipation space can be ensured, and the control board can also be protected to prevent liquid of the host case 10 and so on from damaging the control board 70. In addition, in order to better improve the heat dissipation effect of the control board 70, vent holes may also be formed in the main rack case 11 and/or the main control case 12, and the components on the control board 70 may be cooled down to protect the control board 70.

In an optional embodiment, the anesthesia machine further includes an AGSS module 110, the AGSS module 110 being arranged on a side of the host case 10, and the AGSS module 110 being in communication with the anesthesia ventilation circuit, where the AGSS module 110 refers to an anesthetic gas purification system, and is intended to prevent the contamination of a waste anesthetic gas in the anesthesia machine, avoiding that the waste anesthetic gas causes air contamination in the operating room and thus affects the health of medical personnel.

In an optional embodiment, the anesthesia machine further includes a plug-in module interface 120, the plug-in module interface 120 being arranged on the host case 10 and being configured to be connected to a plug-in box module of the anesthesia machine, and the plug-in box module including a heart rate monitor and an electrocardiograph monitor, and so on, which may be specifically selected according to user requirements. In this embodiment, the plug-in box module is connected to the main control board 60 by means of the plug-in module interface 120 and forwards data obtained thereby to the main control board 60, and the main control board 60 processes the received monitoring data and displays same by the display device 21. The plug-in module interface 120 is arranged on the left side or the right side of the host case 10 to facilitate the fixing and maintenance of the plug-in box module and also facilitate the addition or replacement of the plug-in box module.

In an optional embodiment, the plug-in module interface 120 and the AGSS module 110 are located on the same side, and the plug-in module interface 120 is arranged at an upper end of the AGSS module 110, avoiding that the plug-in box module cannot be installed on the anesthesia machine due to a limited space, and also ensuring the neatness of the appearance of the anesthesia machine. In this embodiment, the anesthetic evaporator 30 is placed on the worktable top 111 at the right side of the control panel 40, and the plug-in module interface 120 and the AGSS module 110 are arranged on the main rack case 11 at the left side of the worktable top 111, so that the plug-in module interface 120, the AGSS module 110 and the anesthetic evaporator 30 can be reasonably and effectively arranged, the balance of an anesthesia machine cart can also be ensured, and the movement of the anesthesia machine cart by the medical personnel with handles at two sides of the worktable top 111 is facilitated.

In an optional embodiment, the anesthesia ventilation circuit includes a breathing circuit 50, a driving gas branch providing a driving gas, a fresh gas branch providing a fresh gas, and a bellows-free gas exchanger isolating the driving gas from a breathing circuit gas. In general, the breathing circuit 50 is provided with a carbon dioxide absorption tank 51, and the driving gas branch and the fresh gas branch are both connected to the breathing circuit 50. The carbon dioxide absorption tank 51 is mainly used for filtering out the carbon dioxide exhaled by the patient, and then re-enters the patient's body by means of the breathing circuit 50 together with the fresh gas and the anesthetic vapor generated by the anesthetic evaporator 30, where the driving gas can be oxygen or air, and the gas flow parameter, the gas concentration parameter, the anesthetic concentration parameter of the anesthesia ventilation circuit and so on may be controlled by means of the control panel 40.

Specifically, the anesthesia ventilation circuit further includes a pressure detection assembly 100, the pressure detection assembly 100 being configured to detect a gas pressure of the anesthesia ventilation circuit. In this embodiment, the pressure detection assembly 100, the control panel 40 and the control board 70 are separately arranged on the host case 10, so that the problem of not completely separating gas and electricity in the anesthesia machine is solved, and the safety of the anesthesia machine in use is improved.

In this embodiment, a clamping groove is formed in a side wall inside the host case 10, and a pipeline on the fresh gas branch is fixed to the side wall inside the host case 10 by means of the clamping groove, where the driving gas branch and the breathing circuit 50 are arranged on the outer side of the host case 10.

Specifically, the pressure detection assembly 100 is arranged in the main control case 12, or the pressure detection assembly 100 is arranged at a position of the main rack case 11 on the worktable top, so as to ensure that the pressure detection assembly 100 and the pipeline on the fresh gas branch can be arranged separately from the control panel 40 and the control board 70, avoiding safety accidents caused by gas-electricity cross contact, and reducing hidden safety hazards.

With the above technical solution, the anesthetic evaporator 30, the main control board 60, the control board 70, the power supply interface 80 and the power supply apparatus 130 can be effectively distributed in the regions of the anesthesia machine according to the heat generated by each component in the anesthesia machine during operation, thereby avoiding mutual interference of the components, namely, the influence of a component with a large amount of heat on a component with a small amount of heat, and also shortening the service life of the component with a small amount of heat; also, it is also possible to arrange different heat dissipation structures according to the amounts of heat of components to meet the heat dissipation requirements of different components, so that the components can operate at a safe temperature, thereby solving the problem of component failure caused by the heat generated by the components centralized together during the operation of the anesthesia machine, or even avoiding the risk of possible fires.

Furthermore, the disclosure further separates the gas-electricity components inside the anesthesia machine, avoiding the problem that gas circuit components fail due to heat accumulation during the operation of electronic components, also avoiding the safety accidents caused by the gas-electricity cross contact, and reducing the hidden safety hazards. The components inside the anesthesia machine may be distributed in the various regions inside the anesthesia machine according to parameter information of the components; for example, the amount of heat generated by the main control board 60 is calculated according to the number of components on the main control board 60, the power of a chip, the working frequency of the chip, the manufacturer information of the chip, the size of a capacitor, the manufacturer information of the capacitor, the size of a resistor, and so on, then the main control board 60 is arranged in the display shell 22, so that the heat dissipation effect of the main control board 60 is ensured, forced heat dissipation using a cooling fan is prevented from affecting the working environment of the operating room, the power consumption of the anesthesia machine and the risk of the main control board 60 failing to dissipate heat due to the failure of the cooling fan, and so on are also reduced, thereby achieving many purposes at one stroke.

In the description of the disclosure, it should be noted that unless otherwise expressly specified and defined, the terms "installation", "connecting" and "connection" should be understood in a broad sense, for example, it may be a fixed connection, a detachable connection, or an integrated connection. It may be a mechanical connection or an electrical connection. It may be a direct connection or an indirect connection by means of an intermediate medium, and may be an internal communication of two elements or an interaction relationship of the two elements. For persons of ordinary skill in the art, specific meanings of the foregoing terms in the disclosure may be understood according to specific situations.

In this disclosure, unless otherwise expressly specified and defined, the expression of the first feature being "above" or "under" the second feature may include direct contact of the first feature and the second feature, and may also include that the first and second features are not in direct contact but are in contact by means of additional features therebetween. Furthermore, the expression the first feature being "over", "above" and "on top of" the second feature may be the case that the first feature is directly above or obliquely above the second feature, or only means that the level of the first feature is higher than that of the second feature. The expression the first feature being "underneath", "below" and "beneath" the second feature may be the case that the first feature is directly below or obliquely below the second feature, or only means that the level of the first feature is less than that of the second feature.

This description above provides many different embodiments or examples that can be used to implement different structures of the disclosure. In order to simplify the description of the disclosure, the components and arrangements of specific examples are described above. They are, of course, merely examples and are not intended to limit the disclosure. Furthermore, the disclosure may repeat reference numbers and/or reference letters in different examples, such repetition being for purposes of simplicity and clarity and not in itself indicative of a relationship between various embodiments and/or arrangements as discussed. In addition, the disclosure provides examples of various specific processes and materials herein, but those of ordinary skill in the art may recognize the application of other processes and/or the use of other materials.

In the description, the explanation with reference to the terms such as "an embodiment", "some embodiments", "exemplary embodiments", "examples", "specific examples", or "some examples" means that specific features, structures, materials, or characteristics described in combination with the embodiment(s) or example(s) are included in at least one embodiment or example of the disclosure. In the description, the illustrative expressions of the above-mentioned terms do not necessarily refer to the same embodiments or examples. Moreover, the specific features, structures, materials, or characteristics described herein may be combined in a suitable manner in any one or more embodiments or examples.

Although the embodiments of the disclosure have been shown and described, it will be appreciated by those of ordinary skill in the art that: various changes, modifications, substitutions and alterations can be made to these embodiments without departing from the principles of the disclosure, the scope of which is defined by the claims.

## Claims

1. An anesthesia machine, wherein the anesthesia machine comprises: a gas source interface (90), an anesthesia ventilation circuit, an anesthetic evaporator (30), a host case (10), a main control board (60), a display apparatus (20) and a power supply interface (80); wherein the host case (10) comprises a main rack case (11) and a main control case (12) arranged above the main rack case (11);
the gas source interface (90) and the power supply interface (80) are arranged on the host case (10), and the anesthetic evaporator (30) is detachably connected to the host case (10);
the anesthesia ventilation circuit connects with the gas source interface (90), and at least part of the anesthesia ventilation circuit is arranged in the host case (10);
the display apparatus (20) includes a display device (21) and a display shell (22) for fixing the display device (21), **characterized in that**
the display apparatus (20) is installed at an upper end of the main control case (12) by means of a display supporting frame; and
the main control board (60) is arranged on a side of the display shell (22) opposite to the display device (21), and the power supply interface (80) electrically connects with the main control board (60) and the display device (21).

2. The anesthesia machine of claim 1, **characterized in that** the gas source interface (90) and the power supply interface (80) are separately arranged.

3. The anesthesia machine of claim 1, **characterized in that** a control panel (40) is arranged on a front side of the host case (10) for adjusting parameter information of the anesthesia ventilation circuit and/or displaying control parameter(s) of the anesthesia ventilation circuit.

4. The anesthesia machine of claim 3, **characterized in that** the anesthetic evaporator (30) is arranged on a left side or a right side of the control panel (40), wherein the gas source interface (90) and the power supply interface (80) are arranged on a back side of the host case (10).

5. The anesthesia machine of claim 3, **characterized in that** the anesthesia machine further comprises:
a control board (70) configured to control power supply for various components of the anesthesia machine, wherein the control board (70) is arranged inside the host case (10),
preferably the control board (70) is arranged on a back side of the host case (10) and below the power supply interface (80).

6. The anesthesia machine of any one of claims 1 to 5, **characterized in that** the anesthesia machine further comprises:
a power supply apparatus (130) arranged on a bottom of an inner side of the host case (10) or on a base (112) below the host case (10), the power supply apparatus (130) being arranged separately from the control board (70) and the control panel (40), preferably the control panel being arranged on the main control case (12).

7. The anesthesia machine of claim 6, **characterized in that** the gas source interface (90) is arranged on the main rack case (11), and/or the power supply interface (80) is arranged on the main control case (12).

8. The anesthesia machine of claim 6, **characterized in that** an upper surface of the main rack case (11) forms a worktable top (111), and the anesthetic evaporator (30) is detachably placed on the worktable top (111).

9. The anesthesia machine of claim 7, **characterized in that** the power supply interface (80) comprises:
an auxiliary output interface (81); and
a power input interface (82) arranged on one side of the auxiliary output interface (81),
preferably the control board (70) is arranged on a back side of the main control case (12), and the auxiliary output interface (81) and the power input interface (82) are both arranged above the control board (70).

10. The anesthesia machine of claim 6, **characterized in that** the control board (70) is arranged in the main rack case (11); or the control board (70) is arranged in the main control case (12).

11. The anesthesia machine of claim 1, **characterized in that** the anesthesia machine further comprises:
an AGSS module (110) arranged on a side of the host case (10), the AGSS module (110) being in communication with the anesthesia ventilation circuit.

12. The anesthesia machine of claim 11, **characterized in that** the anesthesia machine further comprises:
a plug-in module interface (120) arranged on the host case (10),
preferably the plug-in module interface (120) and the AGSS module (110) are located on a same side.

13. The anesthesia machine of claim 8, **characterized in that** the anesthesia ventilation circuit comprises a breathing circuit (50), a driving gas branch and a fresh gas branch, wherein the fresh gas branch is arranged on an inner side of the host case (10), and the driving gas branch and the breathing circuit (50) are arranged on an outer side of the host case (10).

14. The anesthesia machine of claim 13, **characterized in that** the anesthesia ventilation circuit further comprises:
a pressure detection assembly (100) configured to detect a gas pressure of the anesthesia ventilation circuit, wherein the pressure detection assembly (100), the control panel (40) and the control board (70) are separately arranged on the host case (10),
preferably the pressure detection assembly (100) is arranged in the main control case (12), or the pressure detection assembly (100) is arranged on the worktable top (111) of the main rack case (11).

15. The anesthesia machine of claim 1, **characterized in that** a heat dissipation structure is arranged on the display shell (22), and the heat dissipation structure corresponds to the main control board (60) in position,
preferably the heat dissipation structure includes a heat conducting pad, one end of the heat conducting pad abutting against the main control board (60), and the other end of the heat conducting pad abutting against the display shell (22).

## Patentansprüche

1. Ein Anästhesiegerät, das Folgendes umfasst: eine Gasquellen-Bedienungseinheit (90), einen Anästhesie-Beatmungskreislauf, einen Anästhesiegas-Verdampfer (30), ein Hauptgehäuse (10), eine Hauptsteuerplatine (60), eine Anzeigevorrichtung (20) und eine Stromversorgungs-Bedienungseinheit (80); wobei das Hauptgehäuse (10) aus einem Hauptregalgehäuse (11) und einem Hauptsteuergehäuse (12) besteht, das oberhalb des Hauptregalgehäuses (11) angeordnet ist;
die Gasquellen- Bedienungseinheit (90) und die Stromversorgungs- Bedienungseinheit (80) am Hauptgehäuse (10) angeordnet sind, und der Anästhesiegas-Verdampfer (30) abnehmbar mit dem Hauptgehäuse (10) verbunden ist;
der Anästhesie-Beatmungskreislauf mit der Gasquellen- Bedienungseinheit (90) verbunden ist, und mindestens ein Teil des Anästhesie-Beatmungskreislaufs im Hauptgehäuse (10) angeordnet ist;
die Anzeigevorrichtung (20) aus einem Anzeigegerät (21) und einem Anzeigegehäuse (22) zur Fixierung des Anzeigegeräts (21) besteht, mit der Eigenschaft,
dass die Anzeigevorrichtung (20) mittels eines Anzeigestützrahmens am oberen Ende des Hauptsteuergehäuses (12) installiert ist, und
die Hauptsteuerplatine (60) auf einer Seite des Anzeigegehäuses (22) angeordnet ist, die dem Anzeigegerät (21) gegenüberliegt, und die Stromversorgungs-Bedienungseinheit (80) elektrisch mit der Hauptsteuerplatine (60) und dem Anzeigegerät (21) verbunden ist.

2. Anästhesiegerät nach Anspruch 1, **dadurch gekennzeichnet, dass** die Gasquellen-Bedienungseinheit (90) und die Stromversorgungs- Bedienungseinheit (80) separat angeordnet sind.

3. Anästhesiegerät nach Anspruch 1, **dadurch gekennzeichnet, dass** ein Bedienfeld (40) auf der Vorderseite des Hauptgehäuses (10) angeordnet ist, um Parameterinformationen des Anästhesie-Beatmungskreislaufs einzustellen und/oder Steuerparameter des Anästhesie-Beatmungskreislaufs anzuzeigen.

4. Anästhesiegerät nach Anspruch 3, **dadurch gekennzeichnet, dass** der Anästhesiegas-Verdampfer (30) auf der linken oder rechten Seite des Bedienfelds (40) angeordnet ist, wobei die Gasquellen-Bedienungseinheit (90) und die Stromversorgungs-Bedienungseinheit (80) auf der Rückseite des Hauptgehäuses (10) angeordnet sind.

5. Anästhesiegerät nach Anspruch 3, **dadurch gekennzeichnet, dass** die Anästhesiemaschine ferner Folgendes umfasst:
eine Steuerplatine (70), die so konfiguriert ist, dass sie die Stromversorgung für verschiedene Komponenten des Anästhesiegeräts steuert. Sie ist im Inneren des Hauptgehäuses (10) angeordnet,
Vorzugsweise ist die Kontrollkarte (70) auf einer hinteren Seite des Wirtsgehäuses angeordnet (10) und unterhalb der Stromversorgungs-Bedienungseinheit (80).

6. Anästhesiegerät nach einem der Ansprüche 1 bis 5, das ferner Folgendes umfasst:
eine Stromversorgungsvorrichtung (130), die am Boden innerhalb des Hauptgehäuses (10) oder auf einem Sockel (112) unterhalb des Hauptgehäuses (10) angebracht ist und von der Steuerplatine (70) und dem Bedienfeld (40) getrennt ist, wobei das Bedienfeld vorzugsweise am Hauptsteuergehäuse (12) angeordnet ist.

7. Anästhesiegerät nach Anspruch 6, **dadurch gekennzeichnet, dass** die Gasquellen-Bedienungseinheit (90) am Hauptregalgehäuse (11) angeordnet ist und/oder die Stromversorgungs- Bedienungseinheit (80) am Hauptsteuergehäuse (12) angeordnet ist.

8. Anästhesiegerät nach Anspruch 6, mit der Eigenschaft, dass die obere Oberfläche des Hauptregalgehäuses (11) eine Arbeitstischplatte (111) bildet und der Anästhesiegas-Verdampfer (30) abnehmbar auf dieser Arbeitstischplatte (111) platziert ist.

9. Anästhesiegerät nach Anspruch 7, **dadurch gekennzeichnet, dass** die Stromversorgungs-Bedienungseinheit (80) Folgendes umfasst:
eine zusätzliche Output-Schnittstelle (81) und
eine Stromeingangs-Schnittstelle (82), die auf einer Seite der zusätzlichen Output-Schnittstelle (81) angeordnet ist,
vorzugsweise ist die Steuerplatine (70) auf der Rückseite des Hauptsteuergehäuses (12) angeordnet, und die zusätzliche Output-Schnittstelle (81) sowie die Stromeingangs-Schnittstelle (82) sind beide oberhalb der Steuerplatine (70) angeordnet.

10. Anästhesiegerät nach Anspruch 6, **dadurch gekennzeichnet, dass** die Steuerplatine (70) entweder im Hauptregalgehäuse (11) oder im Hauptsteuergehäuse (12) angeordnet ist.

11. Anästhesiegerät nach Anspruch 1, **dadurch gekennzeichnet, dass** sie ferner Folgendes umfasst:
Ein Modul des Anästhesie -Gas -Evakuierungssystems (110), das auf einer Seite des Wirtsgehäuses (10) angeordnet ist, ist das Anästhesie -Gas -Evakuierungssystem (110) in Kommunikation mit der Belüftungskreis d 'Anästhesie

12. Anästhesiegerät nach Anspruch 11, **dadurch gekennzeichnet, dass** sie ferner Folgendes umfasst:
eine Plug-in-Modul-Schnittstelle (120), die am Hauptgehäuse (10) angeordnet ist,
vorzugsweise befinden sich die Plug-in-Modul-Schnittstelle (120) und das AGFS-Modul (110) auf derselben Seite.

13. Anästhesiegerät nach Anspruch 8, **dadurch gekennzeichnet, dass** der Anästhesie-Beatmungskreislauf einen Atemkreislauf (50), einen Treibgasschenkel und einen Frischgasschenkel umfasst, wobei der Frischgasschenkel auf der Innenseite des Hauptgehäuses (10) angeordnet ist und der Treibgasschenkel sowie der Atemkreislauf (50) auf der Außenseite des Hauptgehäuses (10) angeordnet sind.

14. Anästhesiegerät nach Anspruch 13, **dadurch gekennzeichnet, dass** der Anästhesie-Beatmungskreislauf ferner Folgendes umfasst:
eine Druckerfassungseinheit (100), die so konfiguriert ist, dass sie den Gasdruck des Anästhesie-Beatmungskreislaufs erfasst, wobei die Druckerfassungseinheit (100), das Bedienfeld (40) und die Steuerplatine (70) separat am Hauptgehäuse (10) angeordnet sind,
die Druckerfassungseinheit (100) vorzugsweise im Hauptsteuergehäuse (12) angeordnet oder auf der Arbeitstischplatte (111) des Hauptregalgehäuses (11) platziert ist.

15. Anästhesiegerät nach Anspruch 1, **dadurch gekennzeichnet, dass** eine Wärmeableitungsstruktur am Anzeigegehäuse (22) entsprechend der Position der Hauptsteuerplatine (60) angeordnet ist,
vorzugsweise beinhaltet die Wärmeableitungsstruktur ein wärmeleitendes Pad, wobei ein Ende dieses Pads an der Hauptsteuerplatine (60) und das andere Ende an dem Anzeigegehäuse (22) anliegt.

## Revendications

1. Machine d'anesthésie, où la machine d'anesthésie comprend : une interface de source de gaz (90), un circuit de ventilation d'anesthésie, un évaporateur d'anesthésique (30), un boîtier hôte (10), une carte de commande principale (60), un appareil d'affichage (20) et une interface d'alimentation électrique (80) ; où le boîtier hôte (10) comprend un boîtier de rack principal (11) et un boîtier de commande principale (12) agencé au-dessus du boîtier de rack principal (11) ;
l'interface de source de gaz (90) et l'interface d'alimentation électrique (80) sont agencées sur le boîtier hôte (10), et l'évaporateur d'anesthésique (30) est relié de manière détachable au boîtier hôte (10) ;
le circuit de ventilation d'anesthésie est relié à l'interface de source de gaz (90), et au moins une partie du circuit de ventilation d'anesthésie est agencée dans le boîtier hôte (10) ;
l'appareil d'affichage (20) inclut un dispositif d'affichage (21) et une coque d'affichage (22) destinée à fixer le dispositif d'affichage (21), **caractérisée en ce que**
l'appareil d'affichage (20) est installé au niveau d'une extrémité supérieure du boîtier de commande principale (12) au moyen d'un châssis de support d'affichage ;
la carte de commande principale (60) est agencée sur un côté de la coque d'affichage (22) opposé au dispositif d'affichage (21), et l'interface d'alimentation électrique (80) est électriquement reliée à la carte de commande principale (60) et au dispositif d'affichage (21).

2. Machine d'anesthésie selon la revendication 1, **caractérisée en ce que** l'interface de source de gaz (90) et l'interface d'alimentation électrique (80) sont agencées séparément.

3. Machine d'anesthésie selon la revendication 1, **caractérisée en ce qu'**un panneau de commande (40) est agencé sur un côté avant du boîtier hôte (10) afin de régler des informations de paramètre du circuit de ventilation d'anesthésie et/ou d'afficher un ou plusieurs paramètres de commande du circuit de ventilation d'anesthésie.

4. Machine d'anesthésie selon la revendication 3, **caractérisée en ce que** l'évaporateur d'anesthésique (30) est agencé sur un côté gauche ou un côté droit du panneau de commande (40), où l'interface de source de gaz (90) et l'interface d'alimentation électrique (80) sont agencées sur un côté arrière du boîtier hôte (10).

5. Machine d'anesthésie selon la revendication 3, **caractérisée en ce que** la machine d'anesthésie comprend en outre :
une carte de commande (70) configurée pour commander l'alimentation électrique de divers composants de la machine d'anesthésie, où la carte de commande (70) est agencée à l'intérieur du boîtier hôte (10),
de préférence la carte de commande (70) est agencée sur un côté arrière du boîtier hôte (10) et sous l'interface d'alimentation électrique (80).

6. Machine d'anesthésie selon l'une quelconque des revendications 1 à 5, **caractérisée en ce que** la machine d'anesthésie comprend en outre :
un appareil d'alimentation électrique (130) agencé sur une partie inférieure d'un côté intérieur du boîtier hôte (10) ou sur une base (112) sous le boîtier hôte (10), l'appareil d'alimentation électrique (130) étant agencé séparément de la carte de commande (70) et du panneau de commande (40), de préférence le panneau de commande étant agencé sur le boîtier de commande principale (12).

7. Machine d'anesthésie selon la revendication 6, **caractérisée en ce que** l'interface de source de gaz (90) est agencée sur le boîtier de rack principal (11), et/ou l'interface d'alimentation électrique (80) est agencée sur le boîtier de commande principale (12).

8. Machine d'anesthésie selon la revendication 6, **caractérisée en ce qu'**une surface supérieure du boîtier de rack principal (11) forme une partie supérieure de table de travail (111), et l'évaporateur d'anesthésique (30) est placé de manière détachable sur la partie supérieure de table de travail (111).

9. Machine d'anesthésie selon la revendication 7, **caractérisée en ce que** l'interface d'alimentation électrique (80) comprend :
une interface de sortie auxiliaire (81) ; et
une interface d'entrée électrique (82) agencée sur un côté de l'interface de sortie auxiliaire (81),
de préférence la carte de commande (70) est agencée sur un côté arrière du boîtier de commande principale (12), et l'interface de sortie auxiliaire (81) et l'interface d'entrée électrique (82) sont toutes deux agencées au-dessus de la carte de commande (70).

10. Machine d'anesthésie selon la revendication 6, **caractérisée en ce que** la carte de commande (70) est agencée dans le boîtier de rack principal (11) ; ou la carte de commande (70) est agencée dans le boîtier de commande principale (12).

11. Machine d'anesthésie selon la revendication 1, **caractérisée en ce que** la machine d'anesthésie comprend en outre :
un module de système d'évacuation des gaz d'anesthésie (110) agencé sur un côté du boîtier hôte (10), le module de système d'évacuation des gaz d'anesthésie (110) étant en communication avec le circuit de ventilation d'anesthésie.

12. Machine d'anesthésie selon la revendication 11, **caractérisée en ce que** la machine d'anesthésie comprend en outre :
une interface de module de branchement (120) agencée sur le boîtier hôte (10),
de préférence l'interface de module de branchement (120) et le module de système d'évacuation des gaz d'anesthésie sont situés sur un même côté.

13. Machine d'anesthésie selon la revendication 8, **caractérisée en ce que** le circuit de ventilation d'anesthésie comprend un circuit de respiration (50), une branche de gaz d'entraînement et une branche de gaz frais, où la branche de gaz frais est agencée sur un côté intérieur du boîtier hôte (10), et la branche de gaz d'entraînement et le circuit de respiration (50) sont agencés sur un côté extérieur du boîtier hôte (10).

14. Machine d'anesthésie selon la revendication 13, **caractérisée en ce que** le circuit de ventilation d'anesthésie comprend en outre :
un ensemble de détection de pression (100) configuré pour détecter une pression de gaz du circuit de ventilation d'anesthésie, où l'ensemble de détection de pression (100), le panneau de commande (40) et la carte de commande (70) sont agencés séparément sur le boîtier hôte (10),
de préférence l'ensemble de détection de pression (100) est agencé dans le boîtier de commande principale (12), ou l'ensemble de détection de pression (100) est agencé sur la partie supérieure de table de travail (111) du boîtier de rack principal (11).

15. Machine d'anesthésie selon la revendication 1, **caractérisée en ce qu'**une structure de dissipation de chaleur est agencée sur la coque d'affichage (22), et la structure de dissipation de chaleur correspond à la carte de commande principale (60) en position,
de préférence la structure de dissipation de chaleur inclut un tampon de conduction de chaleur, une première extrémité du tampon de conduction de chaleur étant en butée contre la carte de commande principale (60), et l'autre extrémité du tampon de conduction de chaleur étant en butée contre la coque d'affichage (22).
